# EUROPEAN PATENT SPECIFICATION

(11) **EP 1 178 807 B1**
(45) Date of publication and mention of the grant of the patent: **29.09.2004**
(21) Application number: 00920742.4
(22) Date of filing: 25.04.2000
(51) Int. Cl.: A61K 31/58

(54) **USE OF ORG 33245 IN COMBINED INTERMITTENT HORMONE THERAPY AND CONTRACEPTION**
VERWENDUNG VON ORG 33245 IN DER KOMBINIERTEN INTERMITTIERENDEN HORMONTHERAPIE UND EMPFÄNGNISVERHÜTUNG
UTILISATION DE LA ORG 33245 EN THERAPIE HORMONALE ET EN CONTRACEPTION COMBINEE INTERMITTENTE

(30) Priority: 29.04.1999 EP 99201390
(43) Date of publication of application: 13.02.2002
(73) Proprietor: Akzo Nobel N.V., 6824 BM Arnhem (NL)
(72) Inventor: COELINGH BENNINK, Herman, Jan, Tijmen, NL-3971 BE Driebergen (NL); DECKERS, Godefridus, Hermanus, Johanna, NL-5344 HW Oss (NL); DOLS, Paul, Peter, Marie, Antonius, NL-5346 VR Oss (NL); ORLEMANS, Everardus, Otto, Maria, NL-5343 XM Oss (NL); SCHOONEN, Wilhelmus, Gerardus, Eduardus, Joseph, NL-5345 BV Oss (NL)
(74) Representative: van Wezenbeek, Petrus M.G.F.
(86) International application number: PCT/EP2000/003747
(87) International publication number: WO 2000/066129

(56) References cited:
- EP-A- 0 549 041
- WO-A-93/17686
- WO-A-93/21927
- WO-A-94/04156
- WO-A-97/49407
- SCHOONEN W.G.E. ET AL: "Human progesterone receptor A and B isoforms in CHO cells. II. Comparison of binding, transactivation and EDD50 values of several synthetic (anti)progestagens in vitro in CHO and MCF-7 cells and in vivo in rabbits and rats" JOURNAL OF STEROID BIOCHEMISTRY AND MOLECULAR BIOLOGY, vol. 64, no. 3, 1998, pages 157-170, XP002124156 United Kingdom

## Description

The invention pertains to an anti-progestagenic steroid of the 11β-aryl, 17-spiromethylene type. Such antiprogestational compounds are known from EP 549041 and EP 582338. As described, their therapeutic use is associated with several advantages, int.al. in view of a strong activity and high selectivity, in which these compounds are markedly distinct from RU 486, which holds as the reference anti-progestagen in the field.

The invention is particularly concerned with a field of use of anti-progestagens wherein the anti-progestagen is not a daily therapy, but is used intermittently, i.e. not daily or continuously, but in a regimen of administration wherein each administration of anti-progestagen is followed by one or more days without anti-progestagen. More particularly, such an intermittent use will be in conjunction with other medication, such as progestagen-only therapy.

It has now been found that, within the above known group of 11β-aryl, 17-spiromethylene steroids, one compound has a surprisingly better suitability than the others for being administered intermittently. This is the compound satisfying the structural formula I given below, hereinafter referred to as Org 33245:

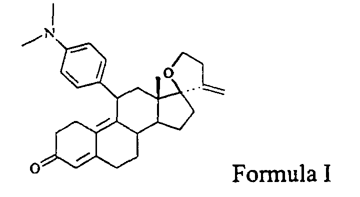

This particular compound therewith has the highly advantageous property that it can be used in the specific medical application of combined therapy with progestagen-only preparations.

Progestagen-only preparations for contraception or HRT (hormone replacement therapy) are known. Contraceptive regimens of this type are usually referred to as "progestagen-only pill' or "POP". Such POPs have the general advantage of avoiding the administration of estrogens. It is known to use anti-progestagens in order to improve the effects of the administration of progestagen-only preparations. This particularly relates to an improved bleeding pattern. Thus major improvements have been proposed, according to which the anti-progestagen is administered periodically, which leads to bleeding patterns that more closely resemble the natural menstrual cycle. One such improvement is that according to Hodgen, see WO 93/21927, wherein a contraceptive regimen free from estrogens is described, in which the active, ovulation-inhibiting ingredient is a progestational agent, and wherein an anti-progestagen is administered intermittently in order to achieve better bleeding (int.al. minimizing progestagen-associated breakthrough bleeding). The anti-progestagen is administered e.g. once every 30, 60, 90, or 120 days, and preferably once every cycle of 30 days (most preferably on day 28 of each cycle). Another such improvement is that according to WO 97/49407, in which it is described to administer, in addition to a progestagen-only preparation, two to seven dosage units comprising an anti-progestagen, one of which is administered at the beginning of a cycle, the other or others divided regularly throughout the cycle (which is described as being 20-32 days and preferably 28).

The concept of a "progestagen only" therapy as indicated above should not be confused with therapies or contraceptive methods in which both the progestagen and the anti-progestagen are administered for a number of consecutive days, as one mlti-day phase in a multiphase regimen. Such a regimen is known from, e.g., WO 94/04156 wherein a contraceptive kit is disclosed which provides a first phase of 5-20 sequential daily dosage units containing an anti-progestagen and a second phase of 10-25 sequential daily dosage units containing a progestagen.

WO 94/04156 describes separate administration of an antiprogestogen for a first period of time (5-20 days) followed by administration of a progestogen for a second period of time (10-25 days), i.e. a two-phase regimen.

Schoonen et al.(1998), J. Steroid Biochem.Mol. Biol. Vol. 64(3), 157-170 describes several anti-progestogens, one of which is Org 33245.

It has been found that, surprisingly, Org 33245 not only has a strong activity and high selectivity, but also shows a strong binding to human orosomucoid, which is indicative of a relatively long half life (Steingold et al. 1990, American Journal of Obstetrics and Gynaecology 162, 532-524). This makes the compound extremely well suitable for intermittent administration, and much better so than anti-progestagens proposed earlier for this use, such as RU 486 and Org 33628.

It should be noted that the excellent suitability of Org 33245 comes all the more as a surprise since this could not be expected from the closely related progestagen Org 33628 which, in fact, has been proposed for use in the regimens described in EP 549041 and EP 582338. Org 33628, although being highly advantageous from the perspective of cost-price and activity, suffers from a drawback particularly associated with intermittent use. This drawback is its relatively rapid metabolism, as can be seen from the short half-life in humans (about 12 hours). This confronts the person skilled in the art with the problem of finding an alternative which has the advantages of Org 33628, but does not have this drawback.

The invention obviates this drawback and provides the use of Org 33245 in the manufacture of a contraceptive or HRT agent wherein Org 33245 is to be administered intermittently, the intermission between each pair of sequentially administered dosage units of anti-progestagen being more than 1 day. The invention particularly is in the use of Org 33245 for the manufacture of a preparation for the intermittent administration thereof in the course of progestagen-only therapy (including contraception). The invention also pertains to a combination comprising a progestagen and an anti-progestagen, wherein the anti-progestagen is Org 33245.

The term "intermittent" should not be confused with the term "non-continuous." A regular, sequential daily administration (in which one administration, e.g. a daily tablet, is naturally followed by e.g. 24 hours of pause until the next daily administration occurs) is not an intermittent administration as defined in the context of the present invention. As used herein, the term "intermittent" should be understood as being related to a "sequential daily administration" in such way that it could be referred to as a "sequential non-daily intermittent administration." I.e., when in a given sequence of days a sequential daily administration means one dosage unit every day of the sequence (e.g. a tablet), then sequential non-daily administration means that each administration is followed by a pause-period comprising at least one day on which no anti-progestagen is administered, and said pause period is followed by another administration of anti-progestagen. In other words, intermittent administration according to the invention requires that the intermission between each pair of sequentially administered anti-progestagen units is more than 1 day. Clearly, with the intermission being 2 days or longer, the problem incurred with Org 33628 and solved with Org 33245 is all the more eminent.
As will be easily understood by the person skilled in the art, it is intended to include in the invention the compound of formula I, as well as prodrugs and precursors thereof, i.e. those closely related compounds the substituents of which are easily metabolised to the active compound according to formula I, or are readily cleaved to such a compound upon being administered. Together with the most regular prodrugs, the invention thus pertains to the compounds satisfying formula II, and pharmaceutically acceptable salts thereof. 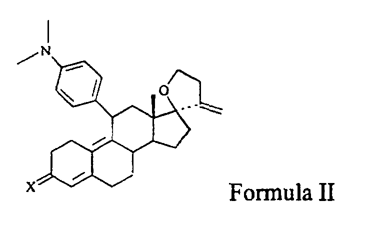 wherein X stands for (H,H), (O), or (N-OH); The 3-keto compound, i.e. Org 33245 itself in which X is (O), is preferred. The other possibilities for the substituent at carbon atom number 3 have as their main property according to the invention that they are precursors (prodrugs) of the preferred 3-keto compound. For the sake of clarity, the invention is described hereinafter with reference to Org 33245 itself.

For the preparation of Org 33245 reference is made to EP 549041 and EP 582338, more specifically Example 1 of EP 549041. In the intermittent use according to the invention, Org 33245 will generally be employed in a dosage amount ranging from 0.1 to 300 mg, and preferably 0.5 to 150 mg. The dosage amount of Org 33245 can be the same each time it is administered, but it may also be used in decreasing amounts as described in WO 97/49407.

The type of administration of Org 33245 can be any type of dosage unit which is suitable for intermittent administration, i.e. it could include an injection which can be given once or several times a month, or it could include a transdermal patch which is applied and removed again once or several times a month, in each case leaving the majority of days without the administration of Org 33245. However, the most convenient and desired form for the intermittent administration of Org 33245 is by way of an oral dosage unit, preferably a tablet.

The intermittent administration of Org 33245 is particularly advantageous in the course of progestagen-only therapy (including contraception). While the anti-progestagen is given intermittently, i.e. on certain days only, it is preferred that on such a day, it is administered together with the progestagen dosage. While an anti-progestagen with a too rapid metabolism will require a precise point in time of administration, and not necessarily simultaneously with the progestagen, Org 33245 can be given in a form physically combined with the progestagen. Thus the invention also includes a combined dosage unit comprising a progestagen and an anti-progestagen, wherein the anti-progestagen is Org 33245.

The invention includes a drug delivery system for contraceptive use (a contraceptive kit) containing daily oral dosage units, each unit containing a progestagen, and 1-7, preferably 1-4 units comprising an anti-progestagen, preferably combined with the progestagen. One of the anti-progestagen dosage units is administered at the end (or, for that matter, the beginning) of a cycle. In fact the anti-progestagen dosage which is given once a cycle, marks the transition from one cycle to the next (i.e. the term "end of the cycle" can be interpreted as the "beginning" of a cycle as well). A second anti-progestagen dosage unit, if given, is administered in the middle of the cycle. If more than two anti-progestagen dosage units are employed, one is given at the end of a cycle, the others orderly divided through the cycle. The preferred dosage regimens are those specifically described in WO 93/21927 and WO 97/49407. The term "cycle" refers to a period of generally 20-35 days, and preferably more close to the natural menstrual cycle, i.e. 28-32 days.

The invention also includes a drug delivery system for HRT (hormone replacement therapy) containing daily oral dosage units, each unit comprising a progestagen with or without an estrogen or an estrogen only, and 1-7, preferably 1-4 dosage units comprising an anti-progestagen, one of which is preferably administered at the beginning of a cycle and the others orderly divided through the cycle (if one other: in the middle of the cycle).

In general terms the invention relates to a contraceptive and/or HRT (hormone replacement therapy) kit comprising sequential daily dosage units for oral administration each comprising as the sole contraceptively effective ingredient a progestagen, or as effective ingredient for HRT a progestagen with or without an estrogen or an estrogen alone, and further two or more units comprising an anti-progestagen.

If desired the kits may contain placebo pills to bridge two periods of administration of active ingredients.

The invention also includes a pharmaceutical product (i.e. the dosage units or the package containing the dosage units), a method of using the product, and a process of manufacturing the pharmaceutical product.

The invention also includes a method of providing contraception and/or HRT for a pre-, peri-, or post-menopausal woman involving administering to the woman the above-mentioned regimens. Thus, the invention also resides in a method of contraception comprising daily administering to a female of child-bearing age a contraceptively effective amount of a progestagen and intermittently administering an anti-progestagen, wherein the anti-progestagen is Org 33245. In another aspect, the invention resides in a method of treatment of irregular or breakthrough bleeding in a female using a progestagen-only preparation, comprising intermittenty administering Org 33245. In these methods it is preferred if Org 33245 is administered on 1-4 days in a cycle of 28-32 days, divided over said cycle, with one of the administrations usually considered the end (or, for that matter, the beginning) of a cycle.

Progestagens for use with the invention are 3-keto-desogestrel (etonogestrel), desogestrel, gestodene, levonorgestel, norgestrel and other progestagens commonly used for contraception and HRT. Desogestrel has the chemical name 13-ethyl-11-methylene-18,19-di-nor-17α-pregn-4-en-20-yn-17-ol, and is the preferred progestagen. Desogestrel is believed to be metabolized in the body into 3-ketodesogestrel. Preferably, the dosage units contain 75 µg of desogestrel or 3-ketodesogestrel, or an amount of other progestagens having the equivalent effect of 75 µg of desogestrel. Based on practically applied doses, levonorgestrel, desogestrel, and 3-keto-desogestrel are relatively equipotent in progestagenic activity. Gestodene is approximately 1.5 times as potent as these compounds. Norgestrel is about half as potent as levonorgestrel. A further preferred progestagen is Org 30659, see int. al. EP 897927.

The invention will be explained further with reference to the following examples.

### Example 1

Org 33245 ((11β,17α)-17,23-epoxy-11-[(4-dimethylamino)phenyl]-19,24-dinorchola-4,9,20-trien-3-one) is synthesized according to Example 1 ofEP 549041.

### Example 2

A range of pharmaceutical compositions is prepared containing a steroid in accordance with the present invention. Org 33245 is mixed with the other ingredients in a standard way, and the mixture is subjected to granulation.
The composition is as follows:

| | |
|---|---|
| Org 33245 (active) | 1-10 wt.%; |
| Corn Starch (disintegrant) | 15 wt.%; |
| Hydroxy Propyl Cellulose (binder) | 3 wt.%; |
| Lactose 200 M (diluent) up to | 100 wt.%; |

The resulting granules can be used for tabletting following procedures regularly available in the art, so as to make a dosage unit suitable for use in the invention.

### Example 3

Of several anti-progestagens the binding to orosomucoid is determined as described in Philibert et al., Antihormones in Health and Disease (M.K. Agarwal, ed.), 1991, 19, 1-17. The results are depicted in the Table below. The results show that, while the binding of the other anti-progestagens tested is lower than that of RU 486 (100%), Org 33245 constitutes a marked improvement.

### Example 4

Anti-progestagenic activity is determined by using an anti-McPhail test as known to the person skilled in the art and described, int.al., in Kloosterboer et al., Human Reproduction (1994), Volume 9, Supplement 1, pages 47-52. Results in terms of the Minimum Active Dose (MAD) are given in the Table below.

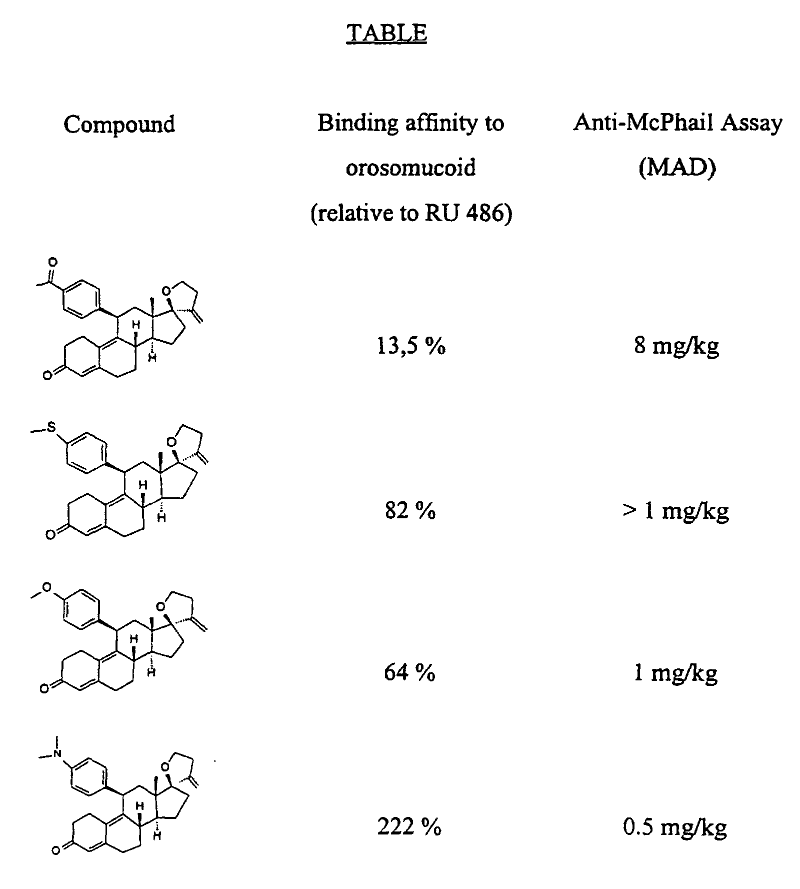

## Claims

1. The use of Org 33245 for the manufacture of a contraceptive or HRT agent wherein Org 33245 is to be administered intermittently, the intermission between each pair of sequentially administered dosage units of anti-progestogen being more than one day.

2. A use according to claim 1, **characterised in that** the intermittent administration of Org 33245 takes place as an addition to progestogen-only-therapy.

3. The use of Org 33245 for the manufacture of a medicament for minimizing uterine bleeding in a female using a progestin-only pharmaceutical preparation, **characterized in that** the anti-progestogen is Org 33245 and is to be administered intermittently, the intermission between each pair of sequentially administered dosage units being more than one day.

4. A use according to any one of the preceding claims, **characterized in that** a dosage of Org 33245 is to be administered 1-7 days during a cycle of 28-32 days, wherein one dosage marks the end of a cycle and the optional other dosages are to be administered regularly divided over the remaining days of the cycle.

5. A contraceptive kit providing means (a) for the daily administration of a progestogen and (b) for the sequential non-daily intermittent administration of an anti-progestogen, wherein the latter means (b) comprises as the anti-progestogen the compound Org 33245.

6. A combined means for the dosage of a progestogen and an anti-progestogen, **characterised in that** the anti-progestogen is Org 33245 and is to be administered intermittently, the intermission between each pair of sequentially administered dosage units being more than one day.

7. A method of contraception comprising daily administering to a female of child-bearing age a contraceptively effective amount of a progestogen and intermittently administering an anti-progestogen, wherein the anti-progestogen is Org 33245 and the intermission between each pair of sequentially administered dosage units of anti-progestogen being more than one day.

8. The use of Org 33245 for the manufacture of a medicament for treating irregular or breakthrough uterine bleeding in a female using a progestin-only preparation, wherein Org 33245 is to be administered intermittently, the intermission between each pair of sequentially administered dosage units of anti-progestogen being more than one day.

9. A method according to claim 7, wherein the anti-progestogen is administered on 1-4 days in a cycle of 28-32 days.

10. A use according to claim 8, wherein the anti-progestogen is administered on 1-4 days in a cycle of 28-32 days.

## Patentansprüche

1. Verwendung von Org 33245 für die Herstellung eines Empfängnisverhütungsmittels oder eines Hormonersatztherapie-Mittels (HRT-Mittels), wobei Org 33245 intermittierend anzuwenden ist, wobei der Zwischenzeitraum zwischen jedem Paar von sequentiell zu verabreichenden Dosiseinheiten von Anti-Progestogen grösser als ein Tag ist.

2. Verwendung nach Anspruch 1, **dadurch gekennzeichnet, dass** die intermittierende Verabreichung von Org 33245 als Zusatz zu einer Nur-Progestogen-Therapie stattfindet.

3. Verwendung von Org 33245 für die Herstellung eines Medikamentes zur Minimierung von Uterusblutungen bei einer Frau, die ein Nur-Progestin pharmazeutisches Präparat einsetzt, **dadurch gekennzeichnet, dass** das Anti-Progestogen Org 33245 ist und intermittierend anzuwenden ist, wobei der Zwischenzeitraum zwischen jedem Paar von sequentiell zu verabreichenden Dosiseinheiten grösser als ein Tag ist.

4. Verwendung nach einem der vorstehenden Ansprüche, **dadurch gekennzeichnet, dass** eine Dosis von Org 33245 1 bis 7 Tage während eines Zyklus von 28 bis 32 Tagen zu verabreichen ist, wobei eine Dosis das Ende eines Zyklus markiert und die optionalen anderen Dosen regelmässig über die verbleibenden Tage des Zyklus verteilt zu verabreichen sind.

5. Ein Verhütungsmittelkit mit Mitteln (a) für die tägliche Verabreichung eines Progestogens und (b) für die sequentielle nicht-tägliche intermittierende Verabreichung eines Anti-Progestogen, wobei das letztere Mittel (b) als Anti-Progestogen die Verbindung Org 33245 umfasst.

6. Ein kombiniertes Mittel für die Dosis eines Progestogens und eines Anti-Progestogens, **dadurch gekennzeichnet, dass** das Anti-Progestogen Org 33245 ist und dass es intermittierend zu verabreichen ist, wobei der Zwischenzeitraum zwischen jedem Paar von sequentiell zu verabreichenden Dosiseinheiten grösser als ein Tag ist.

7. Verfahren zur Empfängnisverhütung mit täglicher Verabreichung an eine Frau im gebärfähigen Alter von einer effektiven Menge eines Progestogens und einer intermittierenden Verabreichung eines Anti-Progestogens, wobei das Anti-Progestogen Org 33245 ist und der Zwischenzeitraum zwischen jedem Paar von sequentiell zu verabreichenden Dosiseinheiten von Anti-Progestogen grösser als ein Tag ist.

8. Verwendung von Org 33245 für die Herstellung eines Medikamentes zur Behandlung von irregulären oder Uterus-Durchbruchblutungen bei einer Frau, die ein Nur-Progestin Präparat einsetzt, wobei Org 33245 intermittierend anzuwenden ist, wobei der Zwischenzeitraum zwischen jedem Paar von sequentiell zu verabreichenden Dosiseinheiten von Anti-Progestogen grösser als ein Tag ist.

9. Verfahren nach Anspruch 7, wobei das Anti-Progestogen 1 bis 4 Tage in einem Zyklus von 28 bis 32 Tagen angewandt wird.

10. Verwendung nach Anspruch 8, wobei das Anti-Progestogen 1 bis 4 Tage in einem Zyklus von 28 bis 32 Tagen angewandt wird.

## Revendications

1. L'utilisation de la Org 33245 pour la fabrication d'un contraceptif ou agent d'hormonothérapie dans lequel la Org 33245 est administrée d'une manière intermittente, l'interruption entre chaque paire d'unités de dosage, administrées séquentiellement, d'anti-progestogène étant de plus d'un jour.

2. Une utilisation selon la revendication 1, **caractérisée en ce que** l'administration intermittente de la Org 33245 est faite en complément d'une thérapie au progestogène seulement.

3. L'utilisation de la Org 33245 pour la fabrication d'un médicament pour minimiser les saignements utérins d'une femelle utilisant une préparation pharmaceutique de progestine seulement, **caractérisée en ce que** l'anti-progestogène est la Org 33245 et qu'il doit être administré de manière intermittente, l'interruption entre chaque paire d'unités de dosage administrées séquentiellement étant de plus d'un jour.

4. Une utilisation selon l'une des revendications précédentes, **caractérisée en ce qu'**une dose de la Org 33245 est administrée 1 à 7 jours durant un cycle de 28 à 32 jours, dans lequel un dosage marque la fin d'un cycle et les autres dosages optionnels sont administrés régulièrement divisés sur la durée des jours restants du cycle.

5. Un kit de contraception fournissant des moyens (a) pour l'administration quotidienne d'un progestogène, et (b) pour l'administration intermittente séquentielle non-journalière d'un anti-progestogène, dans lequel ce dernier moyen (b) comprend comme anti-progestogène le composé Org 33245.

6. Des moyens combinés pour le dosage d'un progestogène et d'un anti-progestogène, **caractérisés en ce que** l'anti-progestogène est la Org 33245 et qu'il doit être administré de manière intermittente, l'interruption entre chaque paire d'unités de dosage administrées séquentiellement étant de plus d'un jour.

7. Une méthode de contraception comprenant l'administration quotidienne à une femelle en âge de procréer, d'une quantité de progestogène efficace comme contraceptif et l'administration intermittente d'un anti-progestogène, dans lequel l'anti-progestogène est la Org 33245 et l'interruption entre chaque paire d'unités de dosage, administrées séquentiellement, d'anti-progestogène étant de plus d'un jour.

8. L'utilisation de la Org 33245 pour la fabrication d'un médicament pour le traitement des saignements utérins irréguliers ou intermenstruels d'une femelle utilisant une préparation de progestine seulement, dans lequel Org 33245 doit être administrée de manière intermittente, l'interruption entre chaque paire d'unités de dosage, administrées séquentiellement, d'anti-progestogène étant de plus d'un jour.

9. Une méthode selon la revendication 7, dans laquelle l'anti-progestogène est administré sur 1 à 4 jours sur un cycle de 28 à 32 jours.

10. Une utilisation selon la revendication 8, dans laquelle l'anti-progestogène est administré sur 1 à 4 jours sur un cycle de 28 à 32 jours.
